# EUROPEAN PATENT APPLICATION

(11) **EP 4 537 895 A1**
(43) Date of publication of application: **16.04.2025**
(21) Application number: 23306774.3
(22) Date of filing: 12.10.2023
(51) Int. Cl.: A61N 5/06, G02C 7/02, G02C 7/10

(54) **OPHTHALMIC SET FOR MYOPIA PROGRESSION CONTROL**

(71) Applicant: Essilor International, 94220 Charenton-Le-Pont (FR)
(72) Inventor: SPIEGEL, Daniel, 424796 Singapore (SG); DROBE, Bjorn, 479251 Singapore (SG); BHANGALE, Sunil Madhukar, 689682 Singapore (SG); KASTURE, Prakhar, 570308 Singapore (SG)
(74) Representative: Korakis-Ménager, Sophie

(57) **Abstract**

the invention relates to ophthalmic set for myopia progression control of a user, which comprises:
- spectral filtering means being configured to selectively emit light in at least one selected range of wavelengths within the visible spectrum of 600 nm to 750 nm when excited by ambient light, allowing retinal exposure of the user myopic eye to said at least one selected range of wavelengths of light, whereby the spectral filtering means are efficient for slowing-down a myopia progression of the user;
- wavefront modifying means adapted to modify wavefronts of the light that enters the user eye for slowing down the myopia progression of the user eye.

## Description

The invention relates to an ophthalmic set for myopia progression control.

In the present description, the terms myopia progression control, myopia progression slowing down, and myopia progression reduction are used with equivalent meanings.

### -- BACKGROUND OF THE INVENTION --

Myopia progression has been established through many observations and well documented for several years, although its cause(s) is (are) still subject to research. Myopia progression is the fact that for one person, his/her myopia increases with time at a rate which almost did not exist before. Kids are the most concerned with myopia progression, and it is thus a major issue to address this problem. Near work and lack of outdoor activities is suspected as being a cause for myopia progression, but the actual biological phenomena and mechanisms which lead to myopia progression remain at hypothesis level.

Several principles and methods have already been proposed for controlling the vision default progression of the myopic eye of a person, including those now mentioned.

One of these methods consists in adding to optical lenses (spectacle lenses or contact lenses) used for correcting the vision of the myopic eye, a plurality of optical elements having a spherical optical function that focus incoming light in front of the retina the eye, in addition to the light crossing the optical lenses out of the plurality of optical elements that is focused on the retina for allowing sharp vision to the wearer who is equipped with these optical lenses or contact lenses.

Another method consists in adding to said optical lenses used for correcting myopia aspherical a plurality of optical elements having a non-spherical optical function.

Still another method consists in adding to the optical lenses used for correcting myopia diffusing or phase-shifting elements which reduce the vision contrast of the person when wearing the optical lenses.

Still other methods implement progressive addition lenses which provide power addition to compensate for the lag of accommodation, or bifocal prismatic lenses for producing both the power addition and a prism effect.

All these methods are based on lenses that are designed for modifying wavefronts of the light that enters the eyes of the wearer or modifying wavefronts of part of this light.

Further methods are based on other principles, such as administering atropine to the subject, or wearing rigid contact lenses during nights for modifying the shape of the cornea.

New solutions are still required to control the myopia progression of wearers.

Studies have been performed recently that suggest a role of the red light in myopia progression control.

The wavelength range from 600nm (nanometer) to 750nm, corresponding to red light is one suspected playing a role for myopia progression control.

Red light therapy suffers several limitations. In particular, the red light is usually delivered at relatively high intensity and this can be bothersome. In addition, some retinal damages due to red light therapy have been recently reported.

It requires to use a specific device with power dependence.

In addition, the different red light therapy protocols impose some timing in a day when the light should be delivered. This can be cumbersome for a wearer and/or the wearer can forget to follow the appropriate protocols.

Starting from this situation, one object of the present invention consists in providing new means for allowing myopia progression control, which are more efficient than those known from prior art.

Another object of the invention is that such means are easy to use for the user, without any specific power-dependent device.

### -- SUMMARY OF THE INVENTION --

For meeting at least one of these objects or others, a first aspect of the present invention proposes an ophthalmic set for a user eye, which comprises:
- spectral filtering means configured to selectively emit light in at least one selected range of wavelengths of light in the visible spectrum of 600 nm to 750 nm when excited by ambient light, preferably in the range of 600 nm to 650 nm, allowing retinal exposure of a wearer eye to said at least one selected range of wavelengths of light;
- wavefront modifying means adapted to modify wavefronts of the light that enters the user eye.

In particular, the ophthalmic set is for myopia progression control of a user eye, and comprises:
- spectral filtering means configured to selectively emit light in at least one selected range of wavelengths of light in the visible spectrum of 600 nm to 750 nm when excited by ambient light, preferably in the range of 600 nm to 650 nm, allowing retinal exposure of a wearer eye to said at least one selected range of wavelengths of light, whereby the spectral filtering means are efficient for slowing-down a myopia progression of the user;
- wavefront modifying means adapted to modify wavefronts of the light that enters the user eye for slowing down the myopia progression of the user eye.

The wavefront modifying means are combined with the spectral filtering means so that the spectral filtering means and the wavefront modifying means are effective simultaneously on the light that enters the user's eye. Thus, respective efficiencies of the wavefront modifying means and spectral filtering means for slowing-down myopia progression are combined for the user of the ophthalmic set. Increased efficiency is thus obtained compared to devices designed only for modifying the light wavefronts.

In some embodiments, spectral filtering means comprise at least a luminescent agent which emits light within the at least one selected range of wavelengths of light within the user eye.

In some embodiments, the luminescence agent is a fluorescent material which emits light by fluorescence in the at least one selected range of wavelengths of light.

In some embodiments, the luminescent agent comprises molecules which absorbs energy in the UV light portion and/or the blue violet light portion of the light spectrum and re emits it in the selected range of wavelengths of light.

In preferred embodiments spectral filtering means are configured to selectively emit light within the at least one selected range of wavelengths of light with an emission rate in the range from 5% to 80%, preferably greater than or equal to 20%.

For some of the preferred embodiments, the spectral filtering means may comprise one among:
- the luminescent agent included in a substrate of an optical lens;
- the luminescent agent included in a film, coating or functional coating adhered on a substrate of an optical lens;
- the luminescent agent included in a patch suitable for being affixed, preferably in a releasable manner, to an optical lens; or
- the luminescent agent included in a clip-on element to be affixed, preferably in a releasable manner, to a spectacle frame in which an optical lens is mounted.

In preferred embodiments, the wavefront modifying means may comprise one of the following:
/i/ an optical lens provided with optical elements having a spherical optical function;
/ii/ an optical lens provided with a plurality of optical elements having a non-spherical optical function;
/iii/ an optical lens provided with a plurality of light-diffusing elements;
/iv/ an optical lens provided with a plurality of phase-shifting elements;/v/ the optical lens of progression addition type or bifocal prismatic type.

In an embodiment, the optical lens is configured for correcting vision of the user myopic eye in said standard wearing conditions, the ophthalmic set further comprises an atropine amount to be administered to the user to be effective when the user is equipped with the wavefront modifying means combined with the spectral filtering means.

In particular, when the wavefront modifying means comprise light-diffusing elements, the ophthalmic set may have one of the following arrangements:
- the spectral filtering means are located on a side of the light-diffusing elements that is opposite to the user, when the ophthalmic set is used; or
- the spectral filtering means are superposed with the light-diffusing elements within a common layer.

Alternatively, when the wavefront modifying means comprise a plurality of optical elements, at least one of said plurality of optical elements may be of refractive type, in particular unifocal or bifocal refractive-type, or diffractive type, in particular pi-Fresnel type.

For others of the preferred embodiments, when the wavefront modifying means comprise a contact lens, the spectral filtering means may comprise one among:
- an optical lens to be worn by the user simultaneously to the contact lens, such optical lens possibly being plano or myopia-correcting; or
- a luminescent agent that is included in a base element of the contact lens.

The ophthalmic set may further comprise at least alert means adapted for informing the user to equip himself with the spectral filtering means, or with the spectral filtering means combined with the wavefront modifying means, at a fixed period, this fixed period may be comprised between 3 to 30 hours per week.

Another aspect of the present invention proposes a method for slowing-down a myopia progression of an eye of a user, in particular of a child, said method comprising providing said user eye with the ophthalmic set defined above.

These and other features of the invention will be now described.

As used herein, a substrate is understood to mean an uncoated substrate, generally with two main faces corresponding in the finished ophthalmic lens to the front and rear faces thereof. The bulk is particularly made of an optical transparent material, generally chosen from transparent materials of ophthalmic grade used in the ophthalmic industry and formed to the shape of an optical lens. The optically transparent material may be a mineral or organic glass.

As used herein, the term coating is understood to mean any layer, layer stack which may be in contact with the optical substrate of the lens and/or with another coating, for example a sol-gel coating or a coating made of an organic resin. A coating may be deposited or formed through various methods, including wet processing, gaseous processing, and film transfer. The functional coatings classically used in optics may be, without limitation, an impact-resistant and/or adhesion primer, an abrasion-resistant and/or scratch-resistant coating, an antireflection coating, an antistatic coating, an anti-soiling coating, an anti-reflective coating, an anti-smudge coating, an anti-dust coating, an anti-fog coating, a water repellent coating, an anti-scratch coating, an interferential filter, a tinted coating, a mirror coating, a photochromic coating, and a combination of any of preceding compatible coatings, especially an impact-resistant primer coating coated with an abrasion and/or scratch-resistant coating.

### -- DETAILED DESCRIPTION OF THE INVENTION --

Although the embodiments of the disclosure are described related to an eyewear, the Man skilled in the art will understand that contact lenses can be used as well.

In the sense of the disclosure, optical lens can be a lens to be mounted in an eyewear or a contact lens.

In one embodiment, an eyewear equipment may comprise a frame designed for fitting the wearer's face, and at least one optical lens.

The optical lens may have a spherical power value suitable for correcting a myopia of the wearer, but the disclosure may also be used for a person devoid of myopia to avoid that such myopia appears and increases later for this person. For such latter case, the lenses do not have spherical power, and are commonly referred to as plano lenses.

According to the invention, this eyewear equipment is intended to slow down a myopia progression that may otherwise concern the wearer.

To this end, the optical lenses comprise spectral filtering means arranged for being effective on light that enters a user's eye, these spectral filtering means being configured to selectively emit light in at least one selected range of wavelengths of light in the visible spectrum of 600 nm to 750 nm when excited by ambient light, preferably in the range of 600nm to 650nm, allowing retinal exposure of a wearer eye to said at least one selected range of wavelengths of light.

Ambient light can be sunlight, artificial light and/or indirect light and/or indoor light.

According to the invention, the spectral filtering means comprise at least one selective optical filter configured to allow selectively substantial emission of at least one selected range of wavelengths of light in the visible spectrum of 600 nm to 750 nm when excited by ambient light, preferably in the range of 600nm to 650nm (herewith defined as the selected range of wavelengths of light or red light).

Substantial emission refers to allowing emission of the selected range of wavelength of light with an average emission rate in the range from 5% to 80%, preferably greater than or equal to 20%.

Indeed, the optical lenses provide an increase of the retinal exposure within the red-light range of the visible spectrum, that have an impact on myopia progression. The lenses with such spectral filtering features are efficient for slowing-down a myopia progression of the user.

The selective optical filter comprises at least a luminescent agent.

As known, the luminescent agent absorbs light energy of a specific wavelength and re-emits light at another wavelength without reflecting substantial amounts of radiation.

In particular, the luminescent agent is configured to receive or absorb ambient light generated by one or more natural or artificial sources and re-emit light within the selected range of wavelengths of light (the red light).

According to several embodiments, the luminescent agent includes a fluorescent material which emits light by fluorescence in the selected range of wavelengths of light.

The chemical nature of the fluorescent material is not particularly limited, provided that it is capable of emitting light by fluorescence, in the red-light range.

The fluorescent materials may be used singly or in combination.

In first exemplary embodiments, the fluorescent material may be a fluorescent molecule that absorbs light in the UV and/or blue violet region of light and re-emits light by fluorescence mainly in the selected range of wavelengths of light.

In different embodiments, fluorescent materials include fluorescent dyes or one or more quantum dots or Q-dots (QDs).

In a non-limitative way, the fluorescent materials comprise fluorescent dyes, in particular chosen from metal complexes of Europium, Terbium, Iridium or Aluminium and any derivatives or combination thereof.

For example, the fluorescent material may comprise Tris(dibenzoylmethane)mono-(phenanthroline)europium(III) dye having a maximum wavelength of emission of 609 nm, Iridium (III) Tris(1-phenylisoquinoline) dye having a maximum wavelength of emission of 613 nm, (dinaphtoylmethane)-mono(phenanthroline)europium (III) dye having a maximum wavelength of emission of 612 nm, Sc-25 and/or Sc-30 Europium dyes.

The quantum dots may be particles that absorbs light in the UV and/or violet region of light and re-emits light mainly in the selected range of wavelengths of light.

For example, the quantum dots are made from or incorporate one or more of the following elements carbon (C), silicon (Si), Cadmium (Cd), germanium (Ge), tin (Sn), lead (Pb), and flerovium (FI) gold or silver QDs,, silicon nanoparticles (Inorganic), Nanocomposite polymers & Phosphor dye doped silica nanoparticules, QDs nanoparticules.

Exemplary materials suitable for use as quantum dots include Cadmium Selenide Zinc Sulfide CdSe/ZnS core-shell type QDs (CdSe/ZnS QDs (DiagNano^{™} COOH CdSe/ZnS QD) having a maximum wavelength of emission of 640 nm ), Carbon QDs (Carbon dot bright Red/7440-44-0) having a maximum wavelength of emission of 600 nm , Cadmium Selenide Cd/se (with a particle size of 7.5 nm) having a maximum wavelength of emission of 650 nm, Silicon quantum dots functionalized) having a maximum wavelength of emission of 610 nm , Silicon QD- Acid terminated (with a particle size 2.5 nm having a maximum wavelength of emission of 650 nm, Silicon Quantum Dots - Dodecyl terminated, Uranine doped Silica Qds having a maximum wavelength of emission of 650 nm.

Advantageously, such fluorescent dyes or QDs allow retina exposure to beneficial red light for myopia progression control.

Spectral filtering features which are used in the invention may be integrated in different ways.

In one embodiment, one or several luminescent agents may be incorporated into the substrate of the optical lenses by being dispersed in a thermoplastic or thermoset polymer material during the manufacture of the substrate itself, for example by casting or injection molding, The luminescent agents can be incorporated into the substrate by methods well known in the art, for example impregnation or imbibition methods consisting in dipping the substrate in an organic solvent and/or water based hot coloration bath, preferably a water based solution, for several minutes.

In another embodiment, the luminescent agent is incorporated into at least one coating coated on the substrate.

In an embodiment, the luminescent agent is incorporated in a layer deposited on the rear face of the optical substrate.

The luminescent agent may be incorporated into any functional coating and, for example, into a primer coating, a hard coating, an abrasion/scratch resistant coating, an antireflection coating, an antistatic coating.

The luminescent agent may be deposited when the layer is prepared from a liquid coating composition or may also be included in a coating in a separate process or sub-process by spin coating, dip coating or spray coating.

The luminescent agent can also be incorporated into a film that will be subsequently transferred, laminated, fused or glued to the substrate or the functional coating.

Still further embodiments of the invention may have one of the following configurations:
- the luminescent agent can be incorporated into a patch suitable for being affixed, preferably in a releasable manner, to the optical lenses or
- the luminescent agent can be incorporated in a clip-on element to be affixed, preferably in a releasable manner, to the frame which accommodates the optical lenses.

The luminescent agents are selected for providing each optical lens with the spectral filtering features defined. The Man skilled in the art knows how to select luminescent agent from spectral features thereof as provided by chemical suppliers and how to adjust concentrations within the material(s) of the lenses for obtaining desired transmission values.

In a first exemplary embodiment, the optical lenses are ORMA^{®} plano lenses substrate having a refractive index of 1.50, a power of (Plano & -2.00 diopters), and a lens thickness of 2 mm.

SC-25 dyes are incorporated into a polymer coating (PU or EPU system) coated on the rear face of the optical lenses. The absorbance of light and subsequent emission of the dyes depends on the dye concentration and coating thickness according to Beer's law.

The details of the exemplary coating are given below:

| **EPU System** | | **PU System** | |
|---|---|---|---|
| **Chemical Name** | **Quantity (g)** | **Chemical Name** | **Quantity (g)** |
| Matrix (EPU Part A) | 3 | Matrix (PU system) | 0.5 |
| Solvent (1-methoxy-2-propanol) | 3.5 | Solvent (Ethylene glycol diacetate) | 1.0 |
| Dye (Sc-25) | 0.03 | Dye (Sc-25) | 0.01 |
| Catalyst (1,8 Diazabicyclo [5,4,0]undec-7-ene | 0.02 | Catalyst (Aluminum Acetyl acetonate) | 0.01 |
| (EPU Part C) | 0.05 | Surfactant (BYK 348) | 0.03 |

Several lens prototypes produced with variation in Sc-25 dyes concentration are defined below:

| **Prototype** | **1** | **2** | **3** | **4** | **5** | **6** | **7** | **8** | **9** |
|---|---|---|---|---|---|---|---|---|---|
| **Type of lens** | Biplano | | | Plano | | | -2 power | | |
| **Dye concentration (%)** | 0.7 | 1 | 1.2 | 0.7 | 1 | 1.2 | 0.7 | 1 | 1.2 |
| **Coating thickness (microns)** | 1.69 | 1.22 | 1.60 | 1.43 | 1.36 | 1.25 | 1.40 | 1.35 | 1.44 |

Measurements of red light intensity, in particular the power of forward emitted fluorescence of the lenses under UV indoor light exposure are given below:

| **Prototypes** | **power over 10mm-port (µW)** | **Fluorescent Intensity (Wm⁻²)** |
|---|---|---|
| UV | 128 | 1,6297 |
| Biplano_01 | 1,312 | 0,0167 |
| Biplano_02 | 2,272 | 0,0289 |
| Biplano_03 | 2,72 | 0,0346 |
| Plano_04 | 0,976 | 0,0124 |
| Plano_05 | 1,664 | 0,0212 |
| Plano_06 | 2,592 | 0,0330 |
| Minus2_07 | 1,024 | 0,0130 |
| Minus2_08 | 1,92 | 0,0244 |
| Minus2_09 | 2,816 | 0,0359 |

In a second exemplary embodiment, the optical lens is coated on front and rear faces by the coating defined in the first embodiment. In such embodiment, the intensity of the red light emitted by the lens is around 1.7 times greater than the intensity of the red emitted light by the lens with the coating on the rear face only.

According to the invention, the spectral filtering means with luminescent agents offer to a wearer a light therapy with continuous and controlled exposure of wearer eye to red light, at low intensity without any specific additional bulky and power-dependent equipment.

In preferred embodiments of the invention, the spectral filtering means recited above are combined within each of the optical lenses with wavefront modifying means also suitable for slowing down myopia progression.

In this way a combined efficiency is obtained for myopia progression slowing down, which is higher than efficiency as resulting from addition of the separated respective effects of the spectral filtering means and wavefront modifying means.

In a preferred embodiment, the wavefront modifying means comprise a plurality of optical elements which are incorporated in one of the lens surfaces, for example its front surface or between the two lens surfaces. When the eyewear is set in worn condition, ie when the optical lens (optical lens or contact lens) is set in front of the wearer eye in standard wearing conditions, each optical element which may be of convergent type, cause the light which crosses it to focus within the eye of the wearer, in front of the wearer's retina. Alternatively, the optical elements may be of non-spherical type, preferably of aspherical type and may have a non -spherical optical function. Advantageously, the optical elements are distributed mainly in a peripheral area of each lens that is disposed in front of the wearer pupil, so that a center area of the lens thereof is substantially free of optical elements and thus provides clear vision to the wearer for the main gaze direction.

In the sense of the disclosure, an "optical function" corresponds to a function providing for each gaze direction the effect of an optical lens on the light ray passing through the optical lens.

In the sense of the disclosure, a "non-spherical optical function" is to be understood as not having a single focus point.

In the sense of the disclosure, a "spherical optical function" is to be understood as having a single focus point.

Advantageously, such optical function of the optical elements reduces the deformation of the retina of the eye of the wearer, allowing to slow down the progression of the abnormal refraction of the eye of the person wearing the lens element.

Advantageously, a part of the plurality of the optical elements is transparent.

Advantageously, the non-contiguous optical elements are not visible on the lens element and do not affect the aesthetic of the lens element.

In the sense of the disclosure, an optical element has a contour shape being inscribable in a circle having a diameter greater than or equal to 0.8 mm and smaller than or equal to 3.0 mm, preferably greater than or equal to 1.0 mm and smaller than 2.0 mm.

According to an embodiment of the disclosure, every circular zone of the optical lens having a radius comprised between 2 and 4 mm comprising a geometrical center located at a distance of the optical center of the optical lens greater or equal to said radius + 5mm, the ratio between the sum of areas of the parts of optical elements located inside said circular zone and the area of said circular zone is comprised between 20% and 70%, preferably between 30% and 60%, and more preferably between 40% and 50%.

The optical elements can be made using different technologies like direct surfacing, molding, casting or injection, embossing, filming, or photolithography etc...

According to an embodiment of the invention, at least one, for example all, of optical elements has a shape configured to create a caustic in front of the retina of the eye of the person. In other words, such optical element is configured so that every section plane where the light flux is concentrated if any, is located in front of the retina of the eye of the person.

According to an embodiment of the invention, at least one of the optical elements, preferably more than 50%, more preferably more than 80% of the optical elements are aspherical microlenses.

In the sense of the invention, aspherical microlenses have a continuous power evolution over their surface.

The aspherical microlenses may have an optical power value higher at their center than their periphery. For example, the variation of optical power over the surface of an aspherical microlens may be comprised between 1.0D and 3.0D in absolute value.

For example, the center of an optical element having a contour shape inscribed in a circle having a diameter equal to 1.2 mm may correspond to a disk having a diameter equal to 0.2 mm centered on the optical center said circle, and the periphery of the micro-lens may correspond to a ring center on the optical center of said circle and having an inner diameter equal to 0.63 mm and an outer diameter equal to 0.73 mm.

According to an embodiment of the invention, the at least one, for example all, of optical element having a non-spherical optical function is a multifocal refractive micro-lens.

In the sense of the disclosure, a "micro-lens" has a contour shape being inscribable in a circle having a diameter greater than or equal to 0.8 mm and smaller than or equal to 3.0 mm, preferably greater than or equal to 1.0 mm and smaller than 2.0 mm.

In the sense of the disclosure, a "multifocal refractive micro-lens" includes bifocals (with two focal powers), trifocals (with three focal powers), progressive addition lenses, with continuously varying focal power, for example aspherical progressive surface lenses.

According to an embodiment of the disclosure, at least one of the optical elements, preferably more than 50%, more preferably more than 80% of the optical elements are aspherical micro-lenses.

In the sense of the disclosure, aspherical micro-lenses have a continuous power evolution over their surface.

The aspherical micro-lenses may have an optical power value greater at their center than at their periphery. For example, the variation of optical power over the surface of an aspherical micro-lens may have an absolute value comprised between 1.0D and 3.0D.

In the sense of the disclosure, the center of the optical element refers to a disk having a diameter comprised between 0.1 mm and 0.5 mm and centered on the optical center of the circle in which the contour shape of the optical element is inscribed, and the periphery of the optical element refers to a ring centered on the center of the circle in which the contour shape of the optical element is inscribed and having an inner diameter comprised between 0.5 mm and 0.7 mm and an outer diameter comprised between 0.70 mm and 0.80 mm

According to an embodiment of the disclosure, the absolute value of the optical power of the aspherical micro-lenses at their center is comprised between 2.0D and 7.0D, and their periphery comprised between 1.5D and 6.0D.

An optical lens which combines both myopia correction and myopia progression control is thus provided. This base optical lens is then covered with a coating which contains the luminescent agent. For example, the coating may be deposited using a dip-coating process, with a dipping solution that contains a blend of coating matrix precursors and luminescent agents. Preferably, such coating may be further designed for providing hard coating properties. Alternatively, the base optical lens may be dipped into a tinting solution which contains the luminescent agent, so that these luminescent agents diffuse into the material of the base. Further tinting processes may also be used, such as dye sublimation.

Possibly, the optical lenses thus provided with both wavefront modifying capability and spectral filtering capability may be further completed with antireflecting coatings and/or hard coatings.

Further embodiments of the invention may be obtained by replacing the optical elements of refractive type as just described, either unifocal, bifocal or aspherical, with other types of optical elements, including of diffractive type, such as pi-Fresnel type.

According to an embodiment of the invention, the central zone of the optical lens corresponding to a zone centered on the optical center of the optical lens does not comprise optical elements. For example, the optical lens may comprise an empty zone centered on the optical center of said lens element and having a diameter equal to 0.9 mm which does not comprise optical elements.

The optical center of the lens element may correspond to the fitting point of the lens. In another preferred embodiment, the wavefront modifying means are light-diffusing elements as described in US 10,302,962. Such light-diffusing elements may be patterns of suitable size which are engraved at the front surface of each optical lens, or small particles of suitable size which are deposited on this front surface. These light-diffusing patterns or particles may be varied in surface-concentration between the peripheral and central areas of the optical lens front surface in order to maintain sharp vision for the main gaze direction. As before, a coating which contains the luminescent agents may be deposited on the lenses, with suitable index difference with respect to the material of the base optical lens or the light-diffusing particles to ensure light-diffusion efficiency. Antireflecting coating and/or hard coating may be further applied on the coating.

Other embodiments of the invention may be obtained by arranging the light-diffusing elements on the rear surface of the optical lens, i.e. on the wearer's side of the spectacle lens or contact lens.

The luminescent agent may be either incorporated within the same layer, or within an additional layer intermediate between the light-diffusing layer and the base optical lens on the rear surface of this latter, or within the substrate itself, encapsulated or within an additional layer which is arranged on the front surface of the lenses.

Further embodiments of the invention may be obtained by combining the spectral filtering means with red light emission with wavefront modifying means comprising optical lenses of progression addition type or bifocal prismatic type.

It is also possible to combine eyewear or contact lenses provided with the spectral filtering means with red light emission with administration of atropine to the wearer and myopia compensation, for obtaining improved myopia progression control.

In other possible embodiments of the invention, the wavefront modifying means may be provided by contact lenses to be worn by the user, and the luminescent agent with red light emission may be incorporated in either the contact lenses, or in lenses of eyewear to be worn in addition to the contact lenses.

The optical set including the spectral filtering means and the wavefront modifying means, may be combined with alert means adapted for informing the user to equip himself with the eyewear at appropriate time. In first possible embodiments of such alert means, they may be comprised of an application hosted in a personal assistant device of the user such as a smartphone. For example, such application may be configured to alert the user to equip himself with the spectral filtering means, or with the spectral filtering means combined with the wavefront modifying means, at a fixed period.

In preferred examples, this fixed period may be comprised between 3 to 30 hours per week.

Invention embodiments different from those described above may be obtained, in particular regarding the actual implementation of the spectral filtering means and/or wavefront modifying means. Using combinations of dyes and interferential filter is possible. Generally, one will understand that combinations of such spectral filtering means with wavefront modifying means of any type may also be used.

## Claims

1. Ophthalmic set for a user eye, which comprises:
- spectral filtering means being configured to selectively emit light in at least one selected range of wavelengths within the visible spectrum of 600 nm to 750 nm when excited by ambient light, allowing retinal exposure of the user eye to said at least one selected range of wavelengths of light;
- wavefront modifying means adapted to modify wavefronts of the light that enters the user eye.

2. Ophthalmic set according to claim 1 wherein the spectral filtering means comprise at least a luminescent agent which emits light within the at least one selected range of wavelengths of light within the user eye.

3. Ophthalmic set according to claim 2, wherein the luminescence agent is a fluorescent material which emits light by fluorescence in the at least one selected range of wavelengths of light.

4. Ophthalmic set according to any of claims 2 to 3, wherein the luminescent agent comprises molecules which absorbs energy in the UV light portion and/or the blue violet light portion of the light spectrum and re emits it in the selected range of wavelengths of light.

5. Ophthalmic set according to any of claims 2 to 4, wherein the spectral filtering means may comprise one among:
- the luminescent agent included in a substrate of an optical lens;
- the luminescent agent included in a film, coating or functional coating adhered on a substrate of an optical lens;
- the luminescent agent is included in a patch suitable for being affixed, preferably in a releasable manner, to an optical lens; or
- the luminescent agent is included in a clip-on element to be affixed, preferably in a releasable manner, to a spectacle frame in which an optical lens is mounted.

6. Ophthalmic set according to any of claims 1 to 5, wherein the wavefront modifying means may comprise one of the following:
/i/ an optical lens provided with optical elements having a spherical optical function;
/ii/ an optical lens provided with a plurality of optical elements having a non-spherical optical function;
/iii/ an optical lens provided with a plurality of light-diffusing elements;
/iv/ an optical lens provided with a plurality of phase-shifting elements;
/v/ an optical lens is of progression addition type or bifocal prismatic type.

7. Ophthalmic set according to claim 6, wherein the optical lens is configured for correcting vision of the user myopic eye in said standard wearing conditions, the ophthalmic set further comprises an atropine amount to be administered to the user so as to be effective when the user is equipped with the wavefront modifying means combined with the spectral filtering means.

8. Ophthalmic set according to any of claims 6 to 7, wherein when the wavefront modifying means comprise light-diffusing elements, the ophthalmic set may have one of the following arrangements:
- the spectral filtering means are located on a side of the light-diffusing elements that is opposite the user, when the ophthalmic set is used; or
- the spectral filtering means are superposed with the light-diffusing elements within a common layer.

9. Ophthalmic set according to any of claims 6 to 8 wherein when the wavefront modifying means comprise a plurality of optical elements, at least one of said plurality of optical elements may be of refractive type, in particular unifocal or bifocal refractive-type, or diffractive type, in particular pi-Fresnel type.

10. Ophthalmic set according to any of claims 1 to 9 further comprising at least alert means adapted for informing the user to equip himself with the spectral filtering means, or with the spectral filtering means combined with the wavefront modifying means.

11. Method for slowing-down a myopia progression of a myopic eye of a user, said method comprising providing said user eye with an ophthalmic set which comprises:
- spectral filtering means being configured to selectively emit light in at least one selected range of wavelengths within the visible spectrum of 600 nm to 750 nm when excited by ambient light, allowing retinal exposure of the user myopic eye to said at least one selected range of wavelengths of light, whereby the spectral filtering means are efficient for slowing-down a myopia progression of the user;
- wavefront modifying means adapted to modify wavefronts of the light that enters the user eye for slowing down the myopia progression of the user eye.
